(19) **Europäisches Patentamt**

**European Patent Office**

**Office européen des brevets**

(11) **EP 1 212 979 A2**

(12) **EUROPEAN PATENT APPLICATION**

(43) Date of publication:
**12.06.2002 Bulletin 2002/24**

(51) Int Cl.$^7$: **A61B 5/022**

(21) Application number: **01128843.8**

(22) Date of filing: **04.12.2001**

| | |
|---|---|
| (84) Designated Contracting States:<br>**AT BE CH CY DE DK ES FI FR GB GR IE IT LI LU**<br>**MC NL PT SE TR**<br>Designated Extension States:<br>**AL LT LV MK RO SI**<br><br>(30) Priority: **06.12.2000 JP 2000371370**<br><br>(71) Applicants:<br>• **Kabushiki Gaisha K-and-S**<br>  **Kariya-shi, Aichi-ken, 448-0001 (JP)**<br>• **KYOHO MACHINE WORKS, Ltd.**<br>  **Toyota-city, Aichi-pref. 471-8515 (JP)**<br><br>(72) Inventors:<br>• **Kondo, Shinji**<br>  **Kariya-shi, Aichi-ken, 448-0001 (JP)** | • **Takemoto, Toru**<br>  **Toyota-city, Aichi-pref. 471-8515 (JP)**<br>• **Honda, Toshihiro**<br>  **Toyota-city, Aichi-pref. 471-8515 (JP)**<br>• **Sakakibara, Noriaki**<br>  **Kariya-shi, Aichi-ken, 448-0001 (JP)**<br><br>(74) Representative:<br>**Leson, Thomas Johannes Alois, Dipl.-Ing.**<br>**Patentanwälte**<br>**Tiedtke-Bühling-Kinne & Partner,**<br>**Bavariaring 4**<br>**80336 München (DE)** |

(54) **Pulse wave measuring apparatus and pulse wave measuring method**

(57)     A maximum blood pressure and a minimum blood pressure are first measured using an oscillometric method. Pulse waves of a vessel occurring during the measurement are measured by a reflection-type photoelectric sensor (13). The maximum and minimum blood pressures and the pulse waves occurring at the times of maximum and minimum blood pressures are associated with each other. After that, the blood pressure is calculated from the associated values and the pulse waves of the vessel serially detected by the reflection-type photoelectric sensor (13).

FIG. 2

EP 1 212 979 A2

## Description

### BACKGROUND OF THE INVENTION

#### 1. Field of the Invention

The invention relates to a pulse wave measuring apparatus and a measuring method thereof.

#### 2. Description of the Related Art

A typical technique of measuring measuring blood pressure pulse waves is a pressure pulse wave vibration method termed an oscillometric method. In this method, after a cuff wrapped around a finger tip or an arm is supplied with air so as to compress the blood vessels, the blood pressure is measured during a decompressing process. On the basis of changes in the values of blood pressure determined by repeating the above-described operation a plurality of times, a pulse wave is measured. However, in the above-described construction, a finger or an arm is repeatedly compressed, and so blood flow is repeatedly stopped in the thus-compressed portion. Therefore, a lengthy measurement may become a burden on the patient. Furthermore, since the blood pressure fluctuates depending on the ambient environment and the internal state of the patient's body, there also is a danger of an inaccurate measurements result due to patient movements.

### SUMMARY OF THE INVENTION

Accordingly, it is an object of the invention to provide a pulse wave measuring apparatus capable of accurately measuring stable pulse waves continuously for a long time without imposing a burden on a patient.

Specifically, an aspect of the invention is a pulse wave measuring apparatus that includes a photoelectric sensor having a light-emitting portion that emits a light to a vessel under the skin of a patient and a light-receiving portion that receives reflected light from the vessel, a blood pressure meter that measures the blood pressure of the patient, and a control portion that measures, as a pulse wave, a time-dependent change of a state of the vessel based on the reflected light.

Furthermore, in the pulse wave measuring method of the invention, light is emitted to a vessel under the skin of a patient, and reflected light from the vessel is received. The blood pressure of the patient is measured. A time-dependent change of a state of the heart or the vessel is measured, as a pulse wave, based on the reflected light.

### BRIEF DESCRIPTION OF THE DRAWINGS

The foregoing and further objects, features and advantages of the invention will become apparent from the following description of preferred embodiments with reference to the accompanying drawings, wherein like numerals are used to represent like elements and wherein:

FIG. 1 is a perspective view illustrating a state where a pulse wave measuring apparatus in accordance with a first embodiment of the invention is attached to a wrist of a patient;

FIG. 2 is a sectional view of the pulse wave measuring apparatus of the first embodiment;

FIG. 3 is a plan view illustrating an arrangement of light-emitting portions and light-receiving portions in accordance with the first embodiment;

FIG. 4 is a block diagram schematically illustrating the electronic layout of the pulse wave measuring apparatus of the first embodiment;

FIGS. 5A and 5B are sectional views of a wrist illustrating a relationship between an arterial vessel and a reflection-type photoelectric sensor of the pulse wave measuring apparatus of the first embodiment;

FIG. 6 is a conceptual diagram indicating results of output of a monitor in accordance with the first embodiment;

FIG. 7 is a schematic illustration of an apparatus for realizing a pulse wave measuring method in accordance with a second embodiment of the invention;

FIG. 8 is a block diagram schematically illustrating an electronic layout of a pulse wave measuring apparatus in accordance with the second embodiment;

FIG. 9 is a flowchart illustrating a blood pressure measuring procedure in the second embodiment;

FIG. 10 is a waveform diagram indicating the cuff pressure and the pressure pulse wave in an oscillometric method;

FIG. 11 is a diagram indicating a blood pressure area;

FIG. 12 is a diagram indicating a pulse wave area;

FIG. 13 is a flowchart illustrating a blood pressure measuring procedure;

FIG. 14 is a flowchart illustrating a pulse wave complementing procedure; and

FIG. 15 is a flowchart illustrating a procedure of calculating the amount of flow of blood.

### DETAILED DESCRIPTION OF PREFERRED EMBODIMENTS

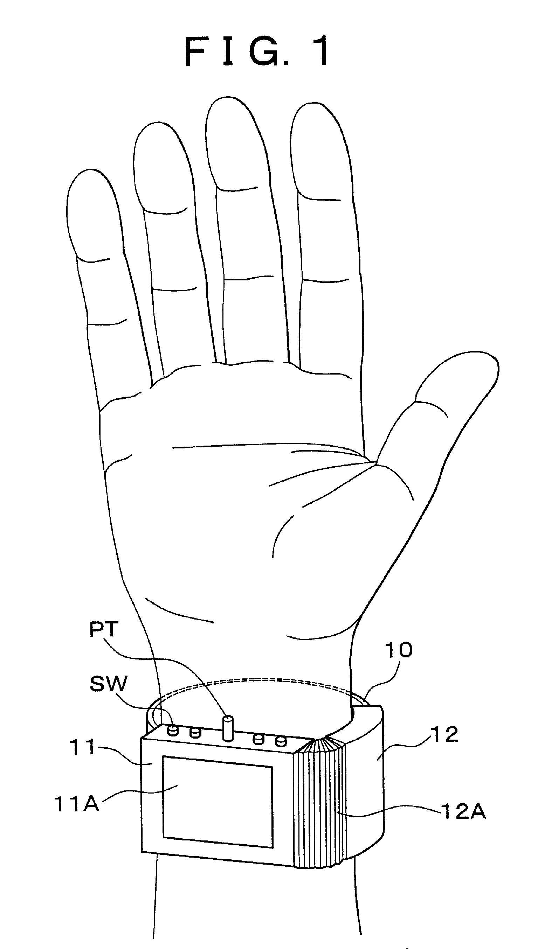

A preferred embodiment of the invention will be described hereinafter with reference to FIGS. 1 to 6. First, a construction of the invention will be described. As shown in FIG. 1, a pulse wave measuring apparatus in accordance with the embodiment has a control portion 11 and a measuring portion 12 in a wristband 10. The wristband 10 has two end portions that are interconnectable, as in a watchband. The entire pulse wave measuring apparatus is wrapped in an annular fashion around a wrist, with the two end portions of the wristband 10 being connected.

The control portion 11 has a flat rectangular parallelepiped shape. The control portion 11 has a monitor 11A on an obverse side, and has in a side surface various operating switches SW and a piston PT for injecting air into a cuff 20 described below.

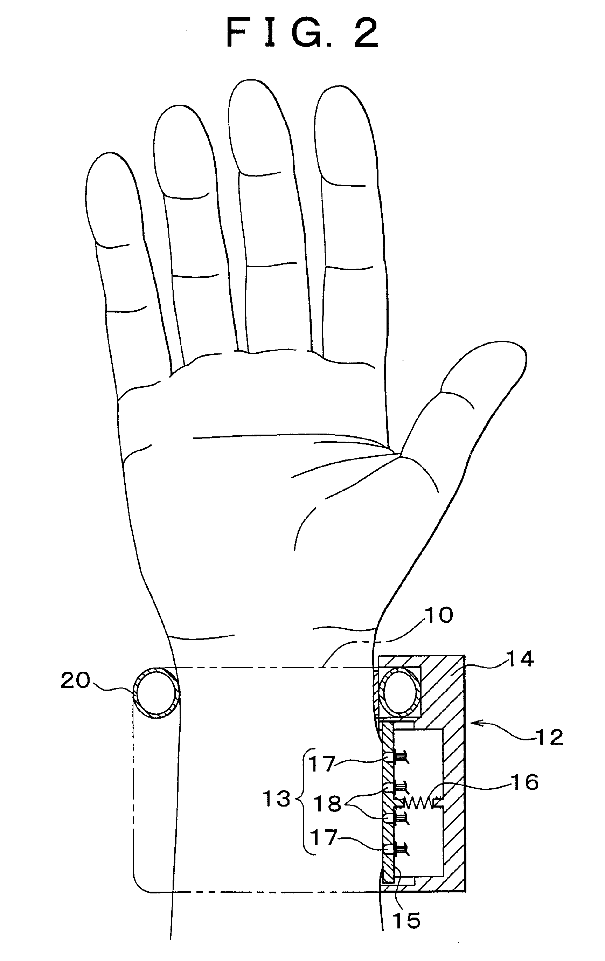

The measuring portion 12 is connected to the control portion 11 via an accordion-like connecting portion 12A. The pulse wave measuring apparatus is attached to a subject's wrist so that the measuring portion 12 contacts a portion of the wrist where an arterial vessel extends. More specifically, as shown in FIG. 2, the measuring portion 12 has a plate member 15 that is disposed within a case portion 14 having an opening in a surface that contacts the wrist. The plate member 15 is slidably mounted at a position at which the plate member 15 closes the opening of the case portion 14 and a receded back wall of the case portion 14. The plate member 15 is always urged toward the opening side of the case portion 14 by a compressed coil spring 16 provided between the plate member 15 and the back wall of the case portion 14. Therefore, the plate member 15 is pressed against a patient's wrist. The elastic restoration force of the compressed coil spring 16 is set to such a magnitude that the blood stream in the arterial vessel will not be stopped.

The plate member 15 has a plurality of through-holes. LEDs as light-emitting portions 17 of a reflection-type photoelectric sensor 13, and photo-diodes as light-receiving portions 18 of the reflection-type photoelectric sensor 13 are fixed to the through-holes of the plate member 15 so as to face toward the opening side of the case portion 14.

A drive circuit for driving the light-emitting portions 17 and a reception circuit for processing output signals of the light-receiving portions 18 are provided in, for example, a control circuit baseboard (not shown) that is disposed in the control portion 11. The reflection-type photoelectric sensor 13 is formed by the drive circuit, the reception circuit, the light-emitting portions 17 and the light-receiving portions 18. The control circuit baseboard is connected with the light-emitting portions 17 and the light-receiving portions 18 by electric cables (not shown) that are excellent in flexibility, so that the plate member 15 can be slid.

In the pulse wave measuring apparatus, the cuff 20 having a tube-like shape extends along an upper peripheral edge portion in FIG. 2. The cuff 20 has an air injection hole in the control portion 11. By using the piston PT (see FIG. 1) protruded from a side surface of the control portion 11, air is injected into the cuff 20. Thus, the cuff 20 is expanded to constrict the entire wrist. The wrist-constricting pressure from the cuff 20 is measured by a pressure sensor 21 that is provided as a blood pressure meter (described below) in the control portion 11.

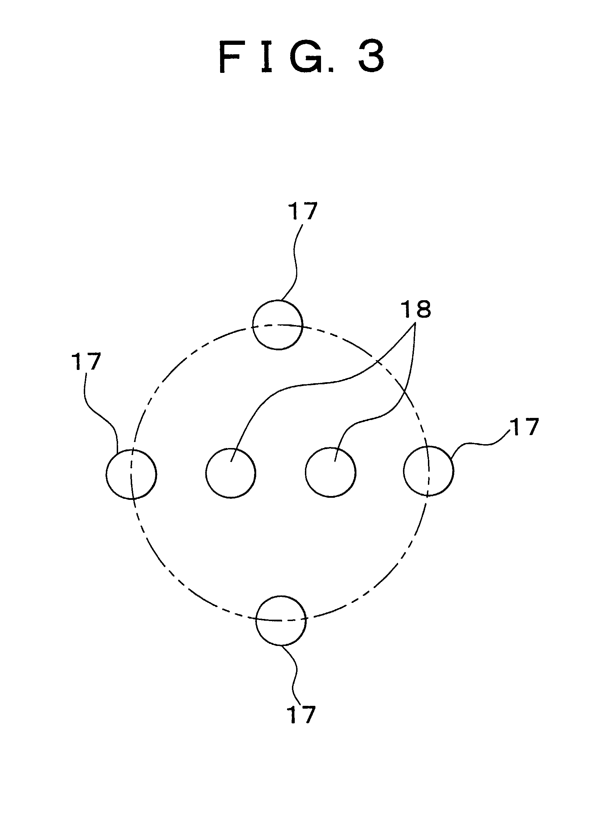

The arrangement of the light-emitting portions 17 and the light-receiving portions 18 is illustrated in detail in FIG. 3. The light-receiving portions 18 are disposed as a pair in a central portion of the plate member 15. The light-emitting portions 17 are disposed at four positions that are equidistantly present on a circle around the light-receiving portions 18. Two of the light-emitting portions 17 are disposed on a line extending through the two light-receiving portions 18.

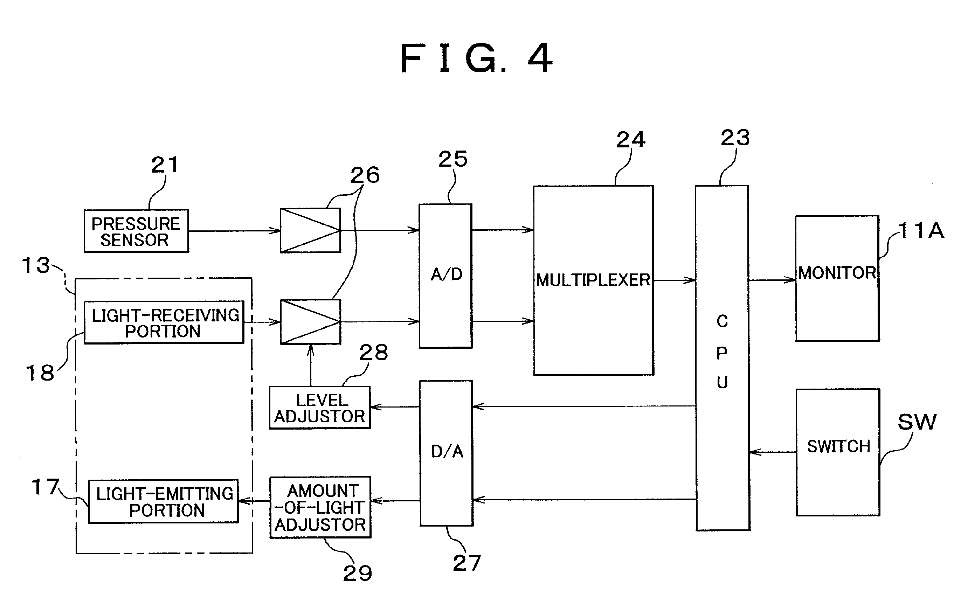

The block diagram of FIG. 4 schematically illustrates the electronic layout of the pulse wave measuring apparatus. As shown in FIG. 4, the pulse wave measuring apparatus has a CPU 23. A multiplexer 24 is connected to an input port of the CPU 23. The pressure sensor 21 and the light-receiving portions 18 are connected to input terminals of the multiplexer 24 via an A/D converter 25 and an amplifier 26. One of an output signal of the pressure sensor 21 and an output signal of the light-receiving portions 18 is appropriately selected by the multiplexer 24, and is inputted to the CPU 23. A result of data processing of the CPU 23 is displayed on the monitor 11A connected to an output port of the CPU 23.

An output port of the CPU 23 is connected to an amount-of-light adjuster 29 and a level adjuster 28 via a D/A converter 27. The amount of light from the light-emitting portions 17 is adjusted based on the output signal of the amount-of-light adjuster 29. The gain of the output amplifier 26 of the light-receiving portions 18 is adjusted by an output signal of the level adjuster 28. The pulse wave measuring apparatus starts operating upon a signal from the switch SW.

Next, an operation of the embodiment constructed as described above will be described. First described will be a method of setting the pulse wave measuring apparatus for operation. The pulse wave measuring apparatus is attached to a wrist or a forearm of a patient by using the wristband 10 in such a manner that the light-emitting portions 17 and the light-receiving portions 18 of the reflection-type photoelectric sensor 13 contact a portion of the wrist or the forearm where an arterial vessel extends (see FIG. 2).

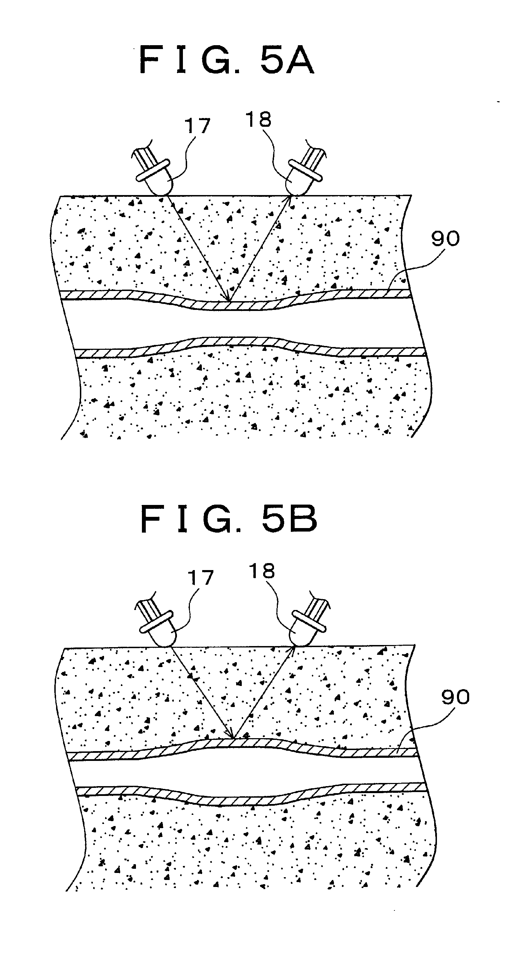

Subsequently, using a switch SW provided on a side surface of the control portion 11, the pulse wave measuring apparatus is powered on. Upon the powering-on operation, the light-emitting portions 17 of the reflection-type photoelectric sensor 13 emit light having a wavelength of 600 to 800 nm to the patient's skin. As shown in FIGS. 5A and 5B, light passes through the patient's skin, and strikes an outer peripheral surface of an arterial vessel 90 in the wrist. Light is reflected from the outer peripheral surface of the arterial vessel 90, and is received by the light-receiving portions 18 of the reflection-type photoelectric sensor 13. In response, the light-receiving portions 18 output a light-reception signal that has a magnitude corresponding to the distance between the reflection-type photoelectric sensor 13 and the arterial vessel 90. The light-reception signal is inputted to the CPU 23 via the amplifier 26 and the like.

As the arterial vessel 90 pulsates in accordance with the pulsation of the heart, the distance between the reflection-type photoelectric sensor 13 and the arterial vessel 90 changes as indicated by FIGS. 5A and 5B. In accordance with such a change in the distance, the amount of light received by the light-receiving portions 18 changes, and therefore, the magnitude of the light-reception signal outputted from the light-receiving portions 18 also changes. The CPU 23 determines that the pulse wave measuring apparatus has been properly attached when, for example, it detects periodical fluctuations of the light-reception signal outputted from the light-receiving portions 18.

Next described will be a procedure of measuring pulse waves. First, an operation instruction, for example, "OPERATE PISTON TO RAISE CUFF PRESSURE" or the like, is displayed on the monitor 11A. When the piston PT is accordingly operated, the cuff 20 is supplied with air, and is gradually inflated. As the air injection into the cuff 20 is continued, the light-reception signal output from the light-receiving portions 18 stops fluctuating. This means a stopped-bloodstream state, that is, a state where the arterial vessel 90 is compressed to stop pulsating by expansion of the cuff 20. The blood pressure present during this state is measured by the pressure sensor 21. The measured value is inputted to the CPU 23 via the amplifier 26 or the like, and is stored as a maximum blood pressure in a memory (not shown). Furthermore, the light-reception signal at the time of measurement of the maximum blood pressure value is also stored in the memory (not shown) as a signal corresponding to the maximum blood pressure. The maximum blood pressure and the signal corresponding to the maximum blood pressure are associated with each other by the CPU 23. After the mode is switched to a continuous measurement mode described below, the CPU 23 is able to calculate a blood pressure value based only on the output signal from the light-receiving portions 18, by using the associated values.

Next, when the force by which the cuff 20 compresses the arterial vessel 90 (hereinafter, referred to as "cuff pressure") is increased to a predetermined value (e.g., about 200 mmHg), an instruction, for example, "END PISTON OPERATION" or the like, is displayed on the monitor 11A. After the piston operation is ended, air is discharged from the cuff 20. As air is discharged, the cuff pressure gradually decreases. During the decrease in the cuff pressure, the light-reception signal output of the light-receiving portions 18 starts to fluctuate again. This means the bloodstream blockage is removed. The blood pressure present at this time is also measured by the pressure sensor 21. The CPU 23 stores a result of the measurement as a minimum blood pressure in the memory. Furthermore, the light-reception signal from the light-receiving portions 18 corresponding to the minimum blood pressure is also stored in the memory. The minimum blood pressure and the light-reception signal corresponding to the minimum blood pressure are associated with each other by the CPU 23.

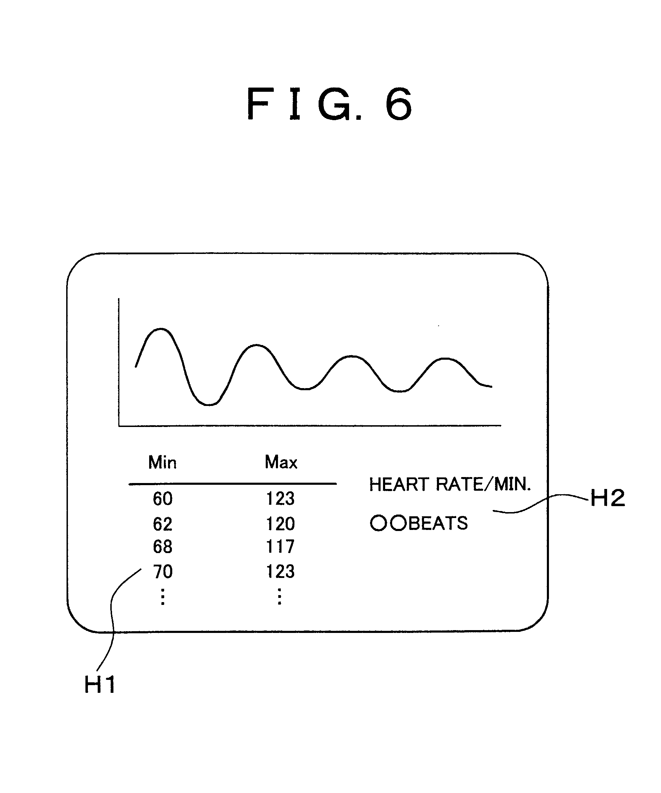

After the maximum blood pressure and the minimum blood pressure are stored in the memory, the pulse wave measuring apparatus, for example, automatically switches to the continuous measurement mode. Then, the light-receiving portions 18 start to continuously detect the pulsation of the arterial vessel 90. As indicated in FIG. 6, time-dependent changes of the light-reception signal outputted from the light-receiving portions 18 are displayed in the form of a graph on the monitor 11A. The CPU 23 determines a maximum value and a minimum value of pulse waves for every heart beat (stroke), and calculates a maximum blood pressure and a minimum blood pressure for every heart beat based on the aforementioned associated values, and display the calculated values on the monitor 11A (as indicated by H1 in FIG. 6). The CPU 23 counts the number of fluctuations of pulse waves measured, and calculates the heart rate in terms of beats per minute, and outputs it on the monitor (as indicated by H2 in FIG. 6). After that, the CPU 23 repeatedly acquires light-reception signals from the light-receiving portions 18, and displays the results of the calculation on the monitor 11A. Then, by checking the graph of the pulse waves, the blood pressure values and the heart rate displayed on the monitor 11A, the physical condition of the patient is diagnosed.

The magnitude or degree of the pulsation of a vessel may greatly vary, for example, between a condition in which a patient is under anesthesia and a condition in which the anesthetic effect has come to an end. Therefore, for example, if the amplification factor of the pulse wave measuring apparatus is fixed in accordance with a state in which the pulse waves are small under anesthesia, the light-reception signal output from the light-receiving portions 18 may become excessively great and the light-reception signal may exceed an appropriate range when the anesthetic effect ends and pulse waves become great. Taking these points into consideration, the pulse wave measuring apparatus of this embodiment is designed so that the CPU 23 drives and controls the amount-of-light adjuster 29 and the level adjuster 28 so that the output signal of the light-receiving portions 18 will not exceed the maximum value and the minimum value of the appropriate range. That is, the CPU 23 drives the amount-of-light adjuster 29 to adjust the amount of light emitted by the light-emitting portions 17 in accordance with the output signal of the light-receiving portions 18. Furthermore, the CPU 23 drives the level adjuster 28 to adjust the amplification factor of the amplifier 26 related to the light-reception signal from the light-receiving portions 18. Therefore, it becomes possible to continuously and stably measure pulse waves from the state where the patient is under anesthesia to the state where the anesthetic effect ends.

According to this embodiment, the compression of a patient's wrist is performed only when the maximum blood pressure and the minimum blood pressure are measured in order to associate the blood pressure and the pulsation with each other. After that, the measurement of the blood pressure is performed by repeatedly detecting pulse waves from the state of pulsation of the vessel by the reflection-type photoelectric sensor 13, and continuously calculating the blood pressure using the associated values. Therefore, the burden on a patient is reduced in comparison with a conventional pulse wave measuring apparatus that repeatedly compresses a vessel. Furthermore, unlike the conventional art in which a vessel is compressed directly by a cuff for detection of pulse waves, the pulse wave measuring apparatus of the embodiment measures pulse waves in an indirect manner based on reflection of light. The pulse wave measurement in accordance with the embodiment is less likely to be affected by movements of a patient, and therefore can be stably performed. Still further, the pulse wave measuring apparatus can be fixed to a wrist or a forearm by the wristband 10, and the blood-pressure meter (the cuff 20, the measuring portion 12, etc.) is integrally provided. Therefore, the operation of associating fluctuations of pulse waves with the maximum blood pressure and the minimum blood pressure can easily be performed.

(SECOND EMBODIMENT)

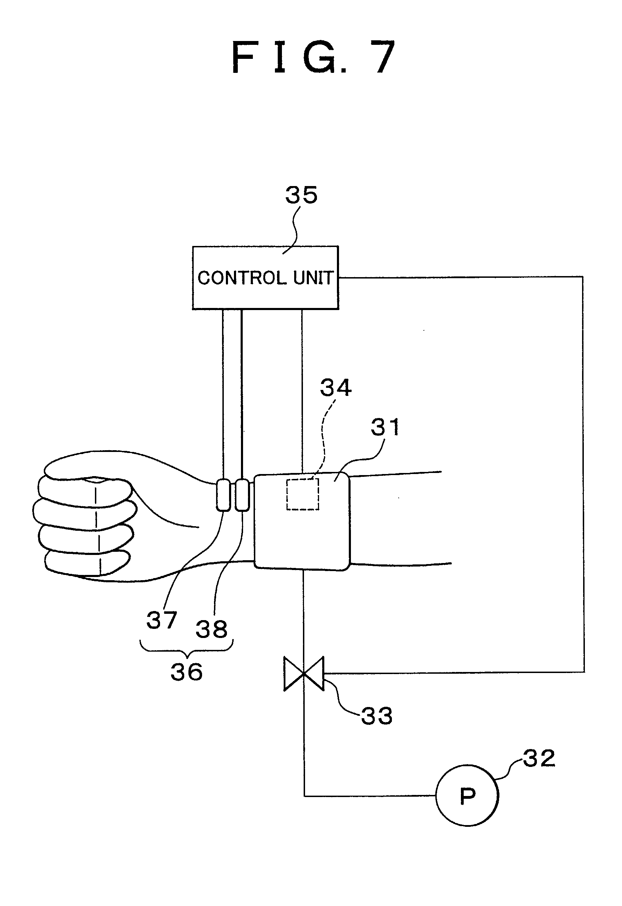

A second embodiment of the invention will be described with reference to FIGS. 7 to 12. FIG. 7 shows a cuff 31 that is attachable to, for example, a wrist. The cuff 31 contains a rubber bag. The rubber bag is connected to an air-supplying pump 32 via a tube. An on-off valve 33 is provided in an intermediate portion of the tube. In accordance with the opening and closing actions of the on-off valve 33, air can be supplied to and discharged from the rubber bag provided in the cuff 31. Further provided within the cuff 31 is a pressure sensor 34 as a blood-pressure meter for detecting fluctuations in air pressure in the rubber bag. The pressure sensor 34 is connected to a control unit 35 provided as a control portion.

A light-emitting sensor 36 is provided at a side of the cuff 31. The light-emitting sensor 36 includes a photoelectric sensor 37 and a body motion sensor 38 disposed adjacent to the photoelectric sensor 37.

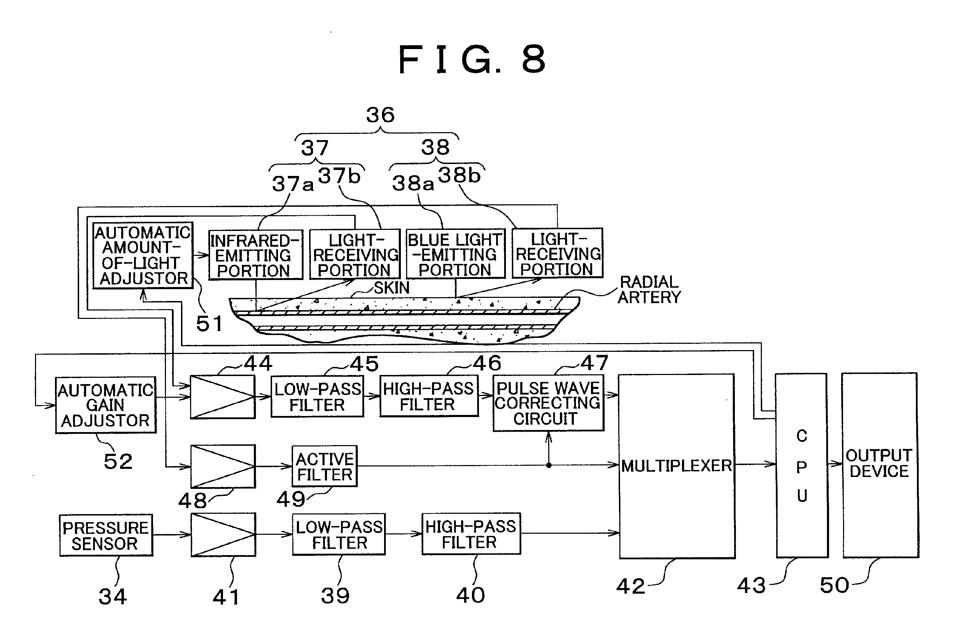

FIG. 8 is a block diagram schematically illustrating an electronic layout of a measuring apparatus in accordance with this embodiment. The photoelectric sensor 37 is formed by a red-light LED (infrared-emitting portion 37a) that emits infrared light having a near-infrared wavelength (e.g., 640 nm) to the skin, and a phototransistor (light-receiving portion 37b) that receives a reflected light. Emitted infrared light passes through a skin surface and reaches a radial artery or a forearm artery located in a deep portion of the skin. Infrared reflected from the radial artery or the forearm artery is received by the light-receiving portion 37b, which outputs a signal. From the output signal of the light-receiving portion 37b, changes in light absorbance caused by volume fluctuations of the radial artery or the forearm artery can be obtained as relative changes in the amount of blood flow.

The body motion sensor 38 is formed by a blue-light LED (blue light-emitting portion 38a) capable of emitting light having an ultra-blue wavelength (e.g., 420 nm) to the skin, and a phototransistor (light-receiving portion 38b) that receives reflected light. Ultra-blue light is reflected from a skin surface, and is received by the light-receiving portion 38b. From an output signal of the light-receiving portion 38b, minute movements of the patient (body motions) detected by the reflected light during the measurement can be attained.

The pressure sensor 34 in the cuff 31 is connected to a low-pass filter 39, and is connected to a high-pass filter 40. Therefore, the output signal from the pressure sensor 34 is processed by the low-pass filter 39 and the high-pass filter 40 so that predetermined frequency components are cut off Then, the output signal is inputted to a CPU 43 via a multiplexer 42. The photoelectric sensor 37 is connected to a high-pass filter 46, and is connected to a pulse wave correcting circuit 47 described below, via an amplifier 44 and a low-pass filter 45. The low-pass filter 45 connected to the photoelectric sensor 37 cuts off a frequency component equal to or lower than 30 Hz in order to remove low-frequency components, including body motions and the like, from the output signal from the light-receiving portion 37b. The high-pass filter 46 is designed so as to cut off a predetermined high-frequency component (equal to or higher than 150 Hz). The body motion sensor 38 is connected to an active filter 49 via an amplifier 48. Of the output signal from the light-receiving portion 38b of the body motion sensor 38, components other than a predetermined frequency band are cut off. As a result, the signal is inputted as a body motion component to the pulse wave correcting circuit 47 and the multiplexer 42.

The pulse wave correcting circuit 47 subtracts a filtered output signal of the body motion sensor 38 from a filtered output signal of the photoelectric sensor 37 so as to generate a waveform obtained by removing a body motion component from the output signal of the photoelectric sensor 37.

The control unit 35 includes the amplifiers 41, 44, 48, the multiplexer 42, the CPU 43, the low-pass filters 39, 45, the high-pass filters 40, 46, the pulse wave correcting circuit 47, the active filter 49, an automatic amount-of-light adjuster 51, and an automatic gain adjuster 52.

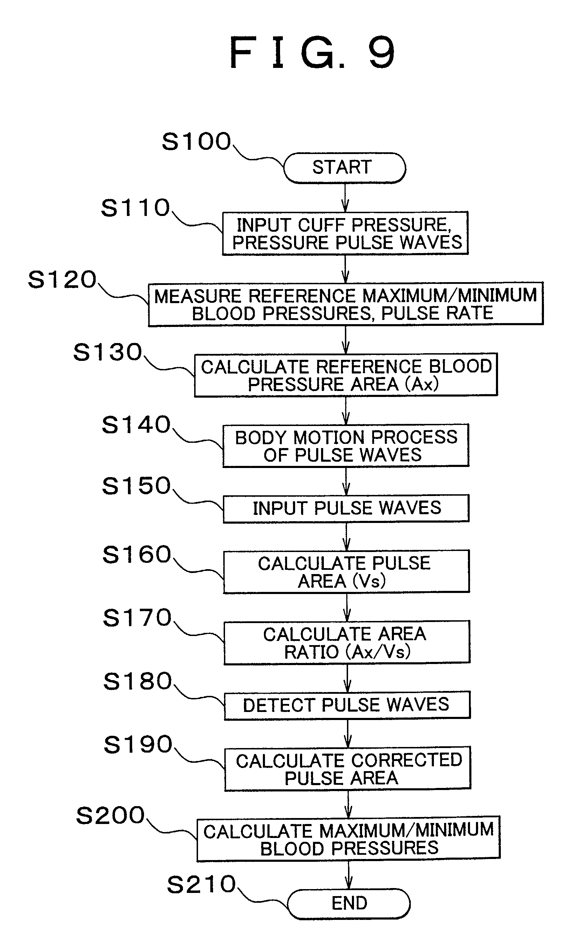

An operation executed by the CPU 43 is illustrated by the flowchart of FIG. 9. First, the pulse wave and the blood pressure are associated with each other for pulse wave measurement. That is, after a cuff pressure (blood pressure) and a pressure pulse wave are inputted (S110), the absolute values of a pulse rate and maximum and minimum blood pressures that serve as references at a predetermined timing are measured based on the cuff pressure and the pressure pulse wave (S120). The maximum and minimum blood pressure values are determined by an oscillometric method.

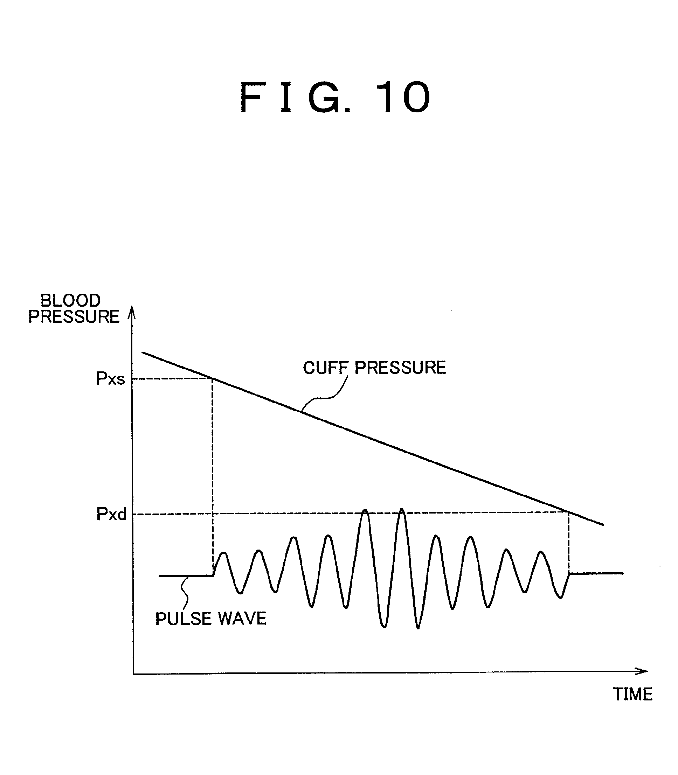

The oscillometric method will be briefly described with reference to FIG. 10. Vibrations of a vessel wall synchronous with beats of the heart are reflected as fluctuations in the cuff pressure (pressure pulse waves). First, the cuff pressure is raised until the pressure pulse waves of a patient disappear (become considerably small). After that, as the cuff pressure is gradually reduced, pressure pulse waves appear (or rapidly become great). The blood pressure occurring at that time is determined as a maximum blood pressure (Pxs in FIG. 10). As the cuff pressure is further reduced, pressure pulse waves disappear again (or rapidly become small). The blood pressure occurring at that time is determined as a minimum blood pressure (Pxd in FIG. 10).

Next, a blood pressure area (reference blood pressure area Ax) at the time of attainment of the reference maximum and minimum blood pressure values is calculated (S130). The reference blood pressure area is determined by an area of a plane figure determined by maximum and minimum blood pressures during a time Tx of a heart beat in a graph with the abscissa axis indicating time and the ordinate axis indicating blood pressure. Specifically, the entire reference blood pressure area (Ax) is determined as a sum of a rectangular region (a lower region Axp2) whose horizontal sides are the beginning and end of a single at heart beat (Tx) and whose top vertical side is the minimum blood pressure (Pxd), and a generally triangular region (an upper region Axp1) whose bottom side has a length corresponding to the heart beat time (Tx) and which has a height corresponding to a difference between the maximum blood pressure and the minimum blood pressure (Pxs - Pxd).

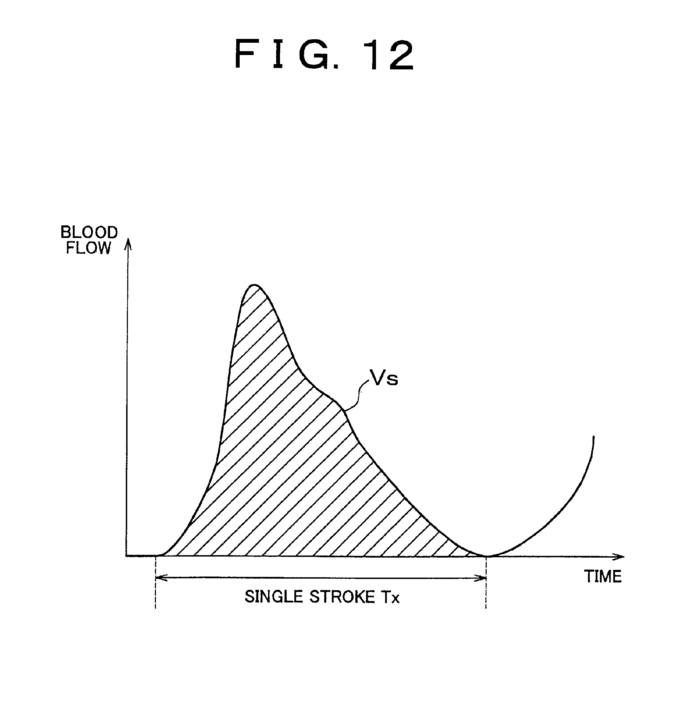

As for the output of the photoelectric sensor 37 (i.e., input of pulse waves) (S150), after execution of a correcting process corresponding to a body motion as described below, a pulse area is calculated based on relative changes in pulse waves outputted from the CPU 43 via a D/A converter (not shown) (S160). Specifically, a reference pulse wave area (Vs) is determined as a value of integration of changes in the amount of blood flow in the heart beat time (Tx) as indicated in FIG. 12. Next, the CPU 43 calculates an area ratio (Ax/Vs) between the reference pulse wave area (Vs) and the reference blood pressure area (Ax) (S170). The thus-obtained area ratio (Ax/Vs) becomes a value in which the pulse wave and the blood pressure are associated with each other. On the basis of the associated value, the infrared-emitting portion 37a of the light-emitting sensor 36 is automatically adjusted in terms of the amount of light by the automatic amount-of-light adjuster 51. Furthermore, the light-receiving portion 37b of the light-emitting sensor 36 is automatically adjusted in terms of gain by the automatic gain adjuster 52. In this manner, the level of the output of the light-emitting sensor 36 is automatically adjusted.

After execution of the gain adjustment and the like as described above, the blood pressure is measured. In this case too, a process for removing noise components such as body motions other than pulse waves is performed. That is, as described above, the output of the photoelectric sensor 37 is processed by the low-pass filter 45 and the high-pass filter 46 so as to provide a waveform in which frequency components of predetermined frequency bands have been removed. At the same time, the output of the body motion sensor 38 is processed by the active filter 49 to remove components such as high-frequency noises and the like. Thus, a waveform of the patient's body motions is obtained. Then, the body motion waveform is subtracted from the pulse wave waveform by the pulse wave correcting circuit 47, so that the pulse waves are corrected (S140).

Then, the pulse area per heart beat (stroke) obtained from the pulse waves continually detected (S180) is multiplied by the area ratio to calculate a corrected pulse area (S190). Subsequently, maximum and minimum blood pressure values for each stroke are continually calculated and determined based on the blood pressure area in accordance with a known blood pressure calculating algorithm (S200).

Next, operation and advantages of the embodiment constructed as described above will be described. First, the cuff 31 is attached to a wrist portion of a patient. Then, the pump 32 is driven to supply air into a rubber bag of the cuff 31. Due to supply of air, the cuff 31 expands to compress a vessel. The supply of air is continued until the compression of the vessel by the cuff 31 results in no pressure pulse wave being detected. At the time point when pulse waves disappear, the air supply to the cuff 31 is stopped. Next, the on-off valve 33 is opened to start reducing the pressure in the cuff 31. Then, the CPU 43 measures the maximum and minimum blood pressure values and the pulses according to the aforementioned oscillometric method.

If the compression state of the vessel varies, accurate measurement may be impeded. Therefore, to make uniform the state of measurement, the measurement is always started in a state where the blood stream is completely blocked by increasing the cuff pressure until there appears no pressure pulse wave. The photoelectric sensor 37 and the body motion sensor 38 emit light of different wavelengths toward the skin. Near infrared light from the infrared-emitting portion 37a of the photoelectric sensor 37 reaches a deep portion of the skin, and is reflected from the radial artery or the forearm artrey. Reflected light is received by the light-receiving portion 37b. Ultra-blue light is emitted from the blue light-emitting portion 38a of the body motion sensor 38 to the skin surface, and reflected light therefrom is received by the light-receiving portion 38b. The received reflected light portions are separately filtered as described above. Then, a pulse area per heart beat is determined, and an associating process, including the automatic gain adjustment and the automatic amount-of-light adjustment with respect to the light-emitting sensor 36, is performed based on the ratio between the pulse area and the blood pressure area determined by the above-described procedure. As a result, the output level of the light-emitting sensor 36 is adjusted.

A state following the aforementioned associating process is an actual measurement stage. The output signal of the photoelectric sensor 37 is processed by the low-pass filter 45 and the high-pass filter 46, so that noises are removed therefrom. Then, via a process of subtracting the output signal from the body motion sensor 38 from the output signal from the photoelectric sensor 37, from which noises have been removed, only pure pulse wave components are successively extracted.

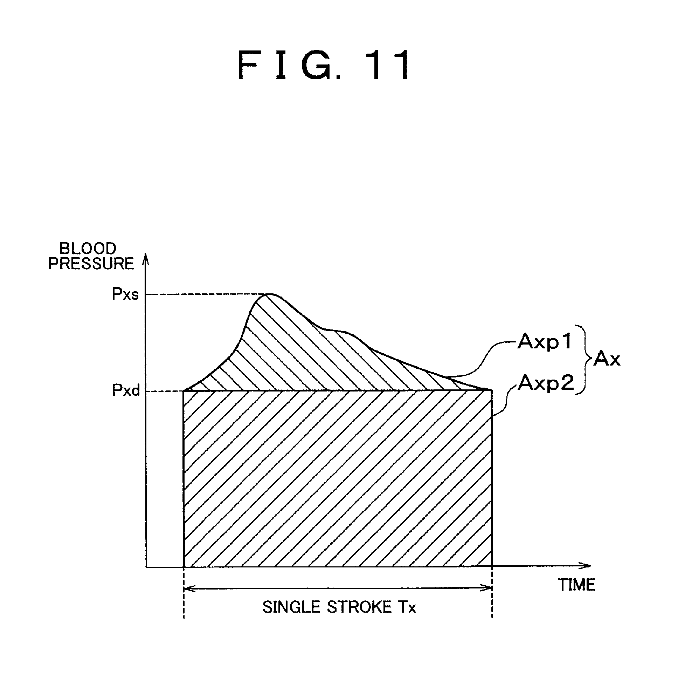

Calculation of the maximum and minimum blood pressure values for each heart beat is performed based on the output signal of the photoelectric sensor 37 that has been subjected to the body motion process, in the following manner. That is, pulse waves and changes in the pressure pulse waves provided by the pressure sensor 34 substantially correspond to each other. For example, the maximum and minimum blood pressure values and the blood pressure area diagram (see FIG. 11) obtained at the reference time may be put in correspondence with corrected pulse areas provided after association at that time. Therefore, if a corrected pulse area at an arbitrary time point is calculated, a blood pressure diagram corresponding to the corrected pulse area can be obtained, and therefore, the maximum and minimum blood pressure values can be determined. Specifically, the maximum and minimum blood pressure values can be determined in the following manner.

It is assumed that Ak is the blood pressure area obtained at the reference time, and that Ax is the blood pressure area in the blood pressure area corresponding to the corrected pulse area diagram obtained at a measurement time point X.

It is also assumed that, at the measurement time point X, the maximum blood pressure is Pxs, and the minimum blood pressure is Pxd, and the area of the upper portion is Axp1, and the area of the lower portion is Axp2, and the heart beat time is Tx.

$$Axp1 = ((Pxs-Pxd)/2) \times Tx \qquad (1)$$

$$Axp2 = Pxd \times Tx \qquad (2)$$

$$Ax = Axp1 + Axp2 \qquad (3)$$

It is also assumed that at the time of reference value measurement, the maximum blood pressure is Pks, and the minimum blood pressure is Pkd, and the area of the upper portion is Axp1, and the area of the lower portion is Axp2, and the heart beat time is Tk.

$$Akp1 = ((Pks-Pkd)/2) \times Tk \qquad (4)$$

$$Akp2 = Pkd \times Tk \qquad (5)$$

$$Ak = Akp1 + Akp2 \qquad (6)$$

Assumed herein are the following equations:

$$Akp1/Akp2 = Ka \qquad (7)$$

$$Axp1/Axp2 = Ka \qquad (8)$$

From the equation (3),

$$Axp2 = Ax - Axp1 \qquad (9)$$

From the equations (9) and (8),

$$Axp1 = (Ka/(1+Ka)) \times Ax \qquad (10)$$

By substitution of the equation (6) in the equation (8),

$$Axp2 = (1/(1+Ka)) \times Ax \qquad (11)$$

From the equation (2),

$$Pxd = Axp2/Tx \qquad (12)$$

By substitution of the equation (11) in the equation (12),

$$Pxd = (1/(1+Ka)) \times (Ax/Tx) \qquad (13)$$

By substitution of the equation (10) in the equation (1),

$$Pxs = ((2Ka/(1+Ka)) \times (Ax/Tx) + Pxd \qquad (14)$$

By substitution of the equation (13 in the equation (14),

$$Pxs = (2Ka+1) \times Ax/((1+Ka) \times Tx) \qquad (15)$$

From the equations (13) and (15), Pxd and Pxs can be determined.

Results of blood pressure measurement attained based on the aforementioned equations are outputted from an output device 50.

As described above, the embodiment is able to continuously measure the blood pressure for each heart beat based on the output signal from the photoelectric sensor 37. Furthermore, since undesired components, such as body motions and the like, can be reliably removed, blood pressure measurement can be accomplished with high precision. Still further, since the ratio between the blood pressure and the pulse area is associated for the measurement, it becomes possible to perform the measurement with less variation despite various physical constitutions of patients.

While the embodiments have been described, the invention is not limited to the above-disclosed embodiments. On the contrary, the technical scope of the invention further includes, for example, embodiments described below. The invention can be modified in various other manners, besides the below-described embodiments, without departing from the spirit of the invention.

Graphs and results of measurement of blood pressure and heart rate may be displayed not only on a monitor attached to the measuring apparatus, and may also be stored in an external storage means.

As for the photoelectric sensors in the embodiment, it is possible to employ infrared sensors, laser light sensors, incandescent lamps, ultraviolet light, etc.

The pulse wave measuring apparatuses of the foregoing embodiments are attachable to a wrist. However, the apparatus of the invention may also be an apparatus attachable to body portions other than the wrist, as long as the apparatus is set to face, via the skin, a vessel that pulsates in response to beats of the heart. In a possible example, a photoelectric sensor is set on the skin of a chest portion, and light is emitted from a light-emitting portion to the heart below the skin. Light reflected from the heart is received by a light-receiving portion provided in a reflection-type photoelectric sensor. Time-dependent changes of the surface position of the heart are measured as pulse waves synchronized with heart beats.

The cuff may also be attached to a subject's upper arm.

The amount of flow of blood may be determined from transition of pulse waves. Specifically, in a possible example, the state of pulsation of a vessel is measured, and a relationship between the state of pulsation and the amount of blood flow is empirically determined. This relationship is stored in a data table. Then, using the CPU, transition of the magnitude of pulse waves is determined, the amount of flow of blood is calculated based on the transition of the magnitude of pulse waves and a value from the data table.

It is also possible to detect the state of pulsation of a vessel based on the transition of pulse waves, and to monitor whether the anesthetic depth has increased or decreased based on expansion or shrinkage of the vessel.

In a further possible example, photoelectric sensors are set at two sites, that is, a forearm and an ankle, and the pulse wave levels at the forearm and the ankle are compared. This will make it possible to detect an abnormality due to constriction of a coronary artery.

If the site of measurement is a wrist, there is a danger of measurement error because the height of the measurement site relative to the heart varies depending on the angle of the arm during measurement. In view of this danger, an angle sensor may be provided, and the CPU may perform an angle compensation.

The light-emitting sensors and the blood pressure meter may be incorporated in the cuff, or may be provided separately from the cuff.

With regard to determination of a blood pressure value, pressure pulse waves changing from appearance to disappearance may be accumulated. The accumulated value may be, for example, averaged in order to determine the maximum and minimum blood pressure value. This will absorb cuff pressure fluctuations during measurement, thereby enhancing the measurement precision.

The arterial cardiac output, the amount of blood flow and the degree of blood oxygen saturation may be measured from a single-heart beat signal attained from pulse waves.

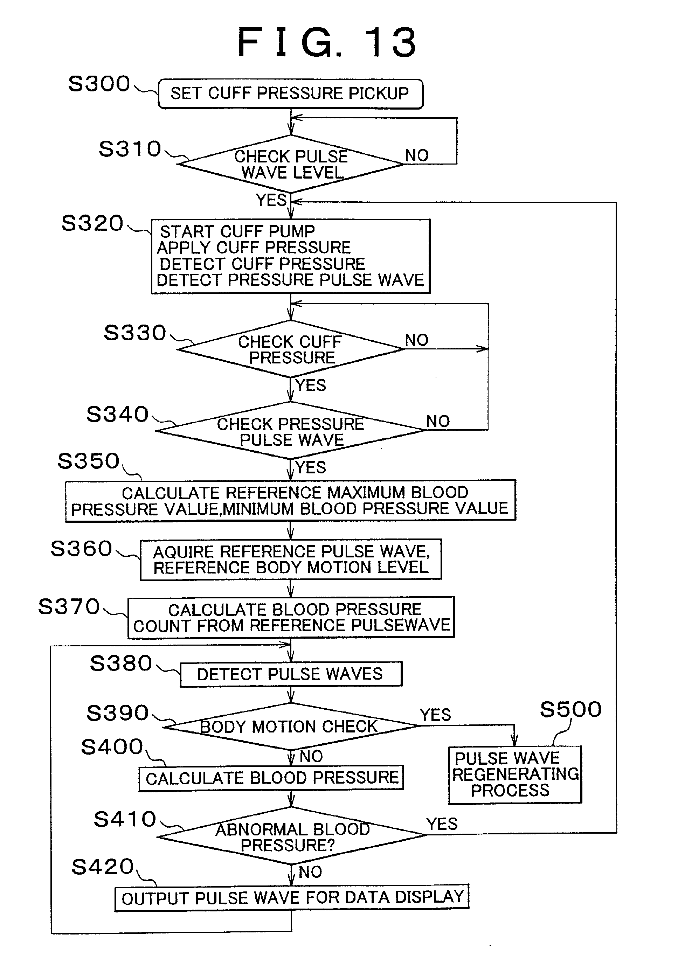

Measurement of blood pressure may be executed following a flowchart shown in FIG. 13. The procedure illustrated by the flowchart will be described, starting at a time point (S300) where the pulse wave measuring apparatus is attached to a patient.

First, it is checked whether the pulse wave level is at an appropriate value, that is, whether the sensor is at an appropriate position with respect to the vessel (S310). If the position is appropriate, the cuff pressure and the pulse waves are detected while the cuff pressure is being raised (S320). The cuff pressure is raised until the cuff pressure reaches a predetermined pressure (S330) and pulse waves disappear (S340). When pulse waves disappear, the pressure and the light reception signal at that time are recorded as a maximum blood pressure and a corresponding light reception signal. Next, the cuff pressure is decreased. The pressure and the light reception signal at the time of appearance of a pulse wave are recorded as a minimum blood pressure and a corresponding light reception signal. After that, the maximum blood pressure and the light reception signal corresponding to the maximum blood pressure are associated with each other, and a reference maximum blood pressure is calculated. Furthermore, the minimum blood pressure and the light reception signal corresponding to the minimum blood pressure are associated with each other, and a reference minimum blood pressure is calculated (S350).

A reference pulse wave and a reference body motion, that is, signals of a state where there is no body motion, are attained via a photoelectric sensor and a body motion sensor (S360). A blood pressure value is calculated from the reference pulse wave (S370).

Subsequently, a pulse wave is detected as a measurement (S380). Next, it is checked whether a body motion has occurred (S390). If no body motion has occurred, a blood pressure value is calculated based on the reference blood pressure and the reference minimum blood pressure calculated as described above (S400). If the obtained blood pressure value is not abnormal (S410), the blood pressure value is displayed in the output device (S420). After that, the process returns to S380, and the process is repeated.

If it is determined that a body motion has occurred in S390, there is a danger of depletion of a pulse wave, so that a pulse wave complementing process is executed (S500).

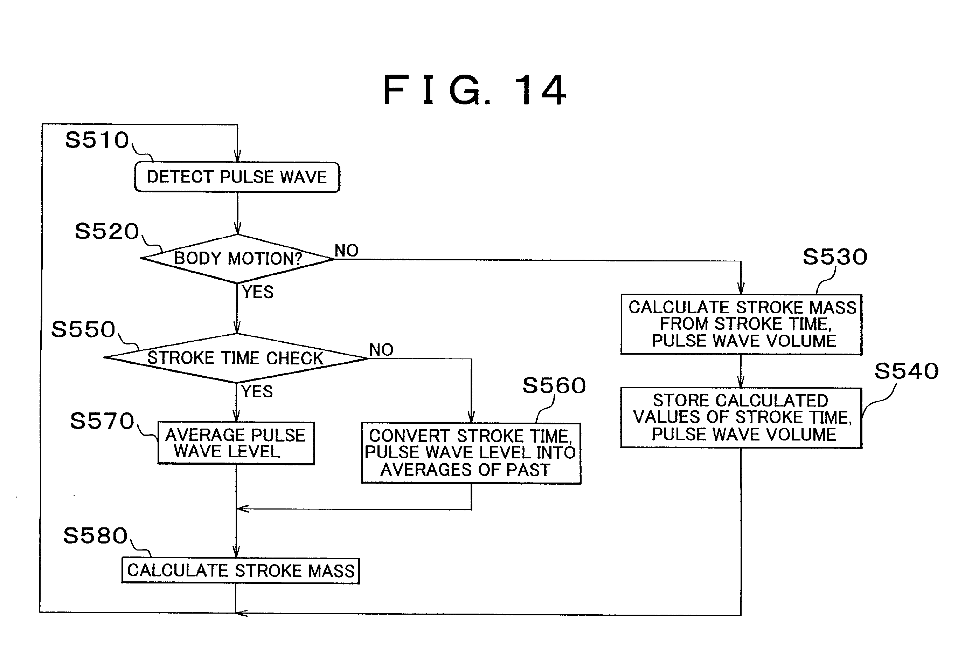

A procedure of complementing pulse waves when a pulse wave measured based on a body motion of a patient or the like has been depleted will be described with reference to FIG. 14.

The pulse wave complementing process is executed following detection of a pulse wave (S510) and determination as to whether a body motion has occurred (S520). If no body motion has occurred, a cardiac output is calculated from the stroke time and the pulse wave volume (S530). The calculated cardiac output is stored in, for example, the control device (S540).

If a body motion has occurred, a stroke time is detected (S550). If a stroke time cannot be detected due to the body motion, the average value of the stroke time and the pulse wave of the past are used (S560) to calculate a cardiac output (S580).

If a body motion has occurred and a stroke time can be checked, only the pulse wave is averaged (S570) to calculate a cardiac output (S580).

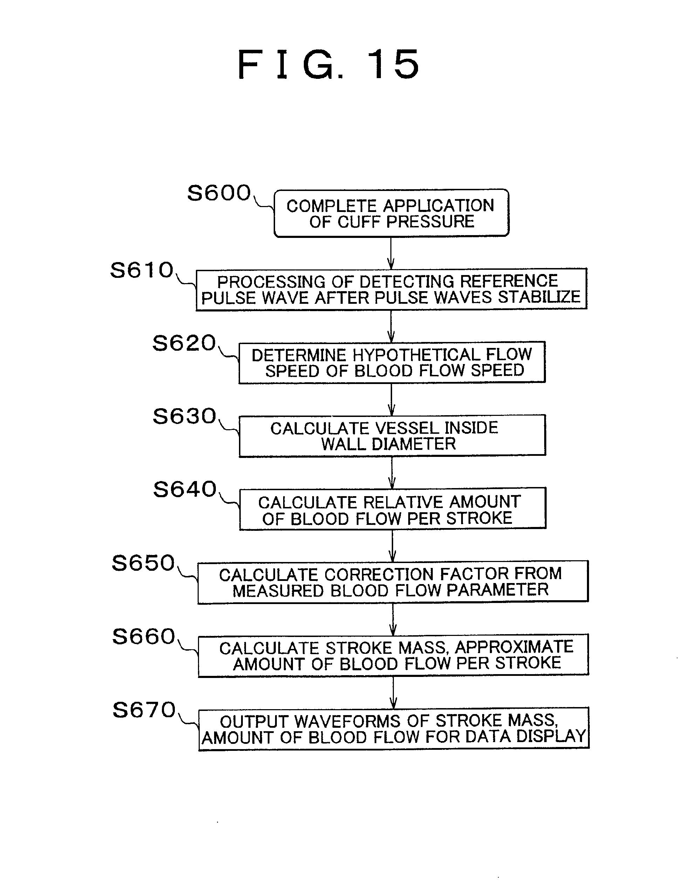

The amount of blood flow may be calculated from the detected pulse wave. The calculating procedure is illustrated by the flowchart of FIG. 15.

The procedure of attaching the measuring apparatus to a patient and applying cuff pressure is the same as described above, and therefore will not be described again. The procedure will be described, starting at a time point when the application of the cuff pressure is completed (S600).

After the pulse waves detected by the measuring apparatus attached to the patient becomes stable, a reference pulse wave detecting process is executed (S610). Next, a hypothetical flow velocity ($v_H$) (e.g., 30 cm/s) of the blood flow velocity (v) is determined (S620).

Next, the inside wall diameter of the vessel is calculated (S630). The inside wall diameter of the vessel can be calculated based on the following relational expression.

$$v_H = \frac{1}{2} \cdot \frac{1}{4h} \cdot \frac{\Delta p}{\Delta l} r^2 \qquad (16)$$

where h is the viscosity of blood, and $\Delta p$ is the pressure difference, and $\Delta l$ is the vessel length, and r is the vessel radius.

Furthermore, a relative amount of flow of blood (Q) per stroke is calculated (S640). The amount of flow of blood can be calculated based on the following equation.

Amount of flow of blood:

$$Q = \frac{1}{\delta h} \cdot \frac{\Delta p}{\Delta l} \cdot \pi r^4 = \pi \cdot r^2 \cdot v_H \qquad (17)$$

Next, a correction factor (k) is calculated (S650). The correction factor can be calculated based on the following equation.

Correction factor:

$$k = \frac{Q}{\Delta p \cdot \pi r^4} \qquad (18)$$

Subsequently, the cardiac output (Co) and an approximate amount of flow of blood per stroke are calculated (S660). The stroke output can be calculated based on the following equation.

Stroke output:

$$Co = hp \cdot Q \qquad (19)$$

where hp is the heart rate per minute.

Finally, the waveforms of the stroke output and the amount of flow of blood obtained as described above are outputted to the output device, such as monitor or the like, for the purpose of data display(S670). Then, this series of processes ends.

The degree of blood oxygen saturation (SaO2) may be calculated and displayed. The blood oxygen saturation can be calculated based on the following equation.

Blood oxygen saturation:

$$SaO2 = (CaO2 - CvO2) \cdot C0x$$

where C0x is the cardiac index, and CaO2 is the amount of oxygen contained in arterial blood, and CvO2 is the amount of oxygen contained in venous blood.

While the invention has been described with reference to what are presently considered to be preferred embodiments thereof, it is to be understood that the invention is not limited to the disclosed embodiments or constructions and is able to detect an eurhythmia and a vein of detail of a body. On the contrary, the invention is intended to cover various modifications and equivalent arrangements.

**Claims**

1. A pulse wave measuring apparatus **characterized by** comprising:

a photoelectric sensor (16, 37) having a light-emitting portion (17, 37a) positionable to emit light to a vessel under a skin of a patient and a light-receiving portion (18, 37b) positionable to receive reflected light from the vessel;
a blood pressure meter (21, 34); and
a control portion (11, 35) adapted to determine a pulse wave based upon a time-dependent change of a state of the vessel based on the reflected light.

2. A pulse wave measuring apparatus according to claim 1, **characterized in that** the control portion (11, 35) is adapted to determine a time-dependent change of a state of a position of a surface of the vessel based on the reflected light.

3. A pulse wave measuring apparatus according to claim 1,**characterized in that** the control portion (11, 35) is adapted to determine a time-dependent change of a state of a photoelectric volume pulse wave of the vessel based on the reflected light.

4. A pulse wave measuring apparatus according to claim 1 to 3, **characterized in:**

**that** the blood pressure meter (21, 34) is adapted to measure a maximum blood pressure and a minimum blood pressure of the patient,
**that** the control portion (11, 35) is adapted to calculates a first value in which the maximum blood pressure and the pulse wave at the time of measurement of the maximum blood pressure are associated, and
**that** the control portion (11, 35) is adapted to calculate a second value in which the minimum blood pressure and the pulse wave at the time of measurement of the minimum blood pressure are associated.

5. A pulse wave measuring apparatus according to claim 4, **characterized in that** the control portion (11, 35) is adapted to calculate the maximum blood pressure and the minimum blood pressure from a maximum value and a minimum value of a newly measured pulse wave for each stroke based on the first value and the second value.

6. A pulse wave measuring apparatus according to claim 1, **characterized by** further comprising a cuff (20, 31) expandable to press the vessel,
wherein the blood pressure meter (21, 34) comprises a pressure sensor (21, 34) positioned to press against the vessel via the skin of the patient upon expansion of the cuff (20, 31), and that determines blood pressure based upon an occurrence of the disappearance of a pressure pulse wave during a process of increasing a pressing force from the cuff (20, 31), and upon the disappearance of a pressure pulse wave again during a process of decreasing the pressing force from the cuff (20, 31).

7. A pulse wave measuring apparatus according to claim 1, **characterized by** further comprising:

a wristband (10) where the photoelectric sensor (13) is mounted; and
an elastic member (16) that is disposed between the photoelectric sensor (13) and the wristband (10) and that produces an elastic force capable of pressing the photoelectric sensor (13) against the vessel via the skin to such an extent that a blood stream is not stopped.

8. A pulse wave measuring apparatus according to claim 7, **characterized by** further comprising:

an angle sensor adapted to detect an inclination angle of an arm with respect to a heart; and
a compensating portion adapted to compensate the pulse wave based on an output of the angle sensor.

9. A pulse wave measuring apparatus according to claim 1, **characterized by** further comprising a body motion-detecting photoelectric sensor (38) having a light-emitting portion (38a) positionable to emit light to the skin of the patient and a light-receiving portion (38b) positionable to receive reflected light from the skin,
wherein the control portion (35) determines a body motion of the patient based on the reflected light from the skin.

10. A pulse wave measuring apparatus according to claim 9, **characterized by** further comprising:

a filter portion (39, 40, 45, 46, 49) that removes a predetermined frequency component from an output signal outputted from the photoelectric sensor (37) and an output signal outputted from the body motion-detecting photoelectric sensor (38); and
a pulse wave correcting portion (47) that corrects the pulse wave for an amount of the body motion based on the output signal from the photoelectric sensor (37) and the output signal from the body motion-detecting photoelectric sensor (38) which have been passed through the filter portion (39, 40, 45, 46, 49).

11. A pulse wave measuring apparatus according to claim 1 to 10, **characterized in that** the control portion (11, 35) is adapted to calculate a heart rate based on the pulse wave.

**12.** A pulse wave measuring apparatus according to claim 1 to 11, **characterized in that** the control portion is adapted to adjust an amount of light outputted from the light-emitting portion (17, 37a) in accordance with the patient.

**13.** A pulse wave measuring apparatus according to claim 1 to 12, **characterized by** further comprising an amplifier portion (27, 44, 48) adapted to amplify a light-reception signal outputted from the light-emitting portion (18, 38b),

wherein the amplifier portion (27, 44, 48) adjusts an amplification factor of the light-reception signal so as to contain the light-reception signal within a predetermined range.

**14.** A pulse wave measuring apparatus according to claim 1 to 3, **characterized in that** the control portion (11, 35) is adapted to calculate an amount of flow of a blood based on the pulse wave.

**15.** A pulse wave measuring apparatus according to claim 1 to 3, **characterized in that** the control portion (11, 35) is adapted to detect a state of the vessel based on the pulse wave, and to measure an anesthetic depth of the patient based on the state of the vessel.

**16.** A pulse wave measuring apparatus according to claim 1, **characterized by** further comprising a second photoelectric sensor having a light-emitting portion positionable to emit light to the vessel under a skin at a position apart from the photoelectric sensor (13, 37), and a light-receiving portion positionable to receives reflected light from the vessel,

wherein the control portion (11, 35) is adapted to diagnose whether there is an abnormality due to a constriction of a coronary artery of the patient by comparing a pulse wave as a time-dependent change of the vessel which is obtained from the reflected light received by the photoelectric sensor (13, 37) and a pulse wave as a time-dependent change of the vessel which is obtained from the reflected light received by the second photoelectric sensor.

**17.** A pulse wave measuring apparatus **characterized by** comprising:

a photoelectric sensor (13, 37) which has a light-emitting portion (17, 37a) that emits a light to a heart under a skin of a chest of a patient and a light-receiving portion (18, 37b) that receives a reflected light from the heart, and which detects a position of a surface of the heart from an amount of the reflected light;
a blood pressure meter (21, 34) that measures a blood pressure of the patient; and

a control portion (11, 35) that measures, as a pulse wave, a time-dependent change of the position of the surface of the heart based on the reflected light.

**18.** A pulse wave measuring method **characterized by** comprising:

emitting a light to a vessel under a skin of a patient;
receiving a reflected light from the vessel; and
determining, a time-dependent change of a state of the vessel based on the reflected light as a pulse wave.

**19.** A pulse wave measuring method according to claim 18, **characterized in that** a time-dependent change of a state of a position of a surface of the vessel is determing based on the reflected light.

**20.** A pulse wave measuring method according to claim 18 to 20, **characterized in that** a time-dependent change of a state of a photoelectric volume pulse wave of the vessel is determined based on the reflected light.

**21.** A pulse wave measuring method according to claim 18, **characterized by** further comprising:

measuring a maximum blood pressure and a minimum blood pressure of the patient;
calculating a first value in which the maximum blood pressure and the pulse wave at the time of measurement of the maximum blood pressure are associated; and
calculating a second value in which the minimum blood pressure and the pulse wave at the time of measurement of the minimum blood pressure are associated.

**22.** A pulse wave measuring method according to claim 21, **characterized in that** the maximum blood pressure and the minimum blood pressure during each stroke are calculated from a maximum value and a minimum value of a newly measured pulse wave for each stroke based on the first value and the second value.

**23.** A pulse wave measuring method according to claim 21, **characterized by** further comprising:

applying a pressing force to the vessel; and
determining a blood pressure occurring when a pressure pulse wave disappears during a process of increasing the pressing force, and a blood pressure occurring when a pressure pulse wave appears during a process of decreasing the pressing force.

**24.** A pulse wave measuring method according to claim 23, **characterized in that** the pulse wave detected at a wrist of the patient is compensated based on a positional relationship between the wrist and the heart.

**25.** A pulse wave measuring method according to claim 18, **characterized by** further comprising emitting a light to the skin of the patient, and measuring a body motion of the patient based on a reflected light from the skin.

**26.** A pulse wave measuring method according to claim 25, **characterized by** further comprising:

removing a predetermined frequency component from a signal obtained from the reflected light; and
performing a correction for an amount of the body motion based on a result of removal of the predetermined frequency component.

**27.** A pulse wave measuring method according to claim 18 to 26, **characterized in that** a heart rate is calculated based on the pulse wave.

**28.** A pulse wave measuring method according to claim 18 to 27, **characterized in that** an amount of the light is adjusted in accordance with the patient.

**29.** A pulse wave measuring method according to claim 18 to 28, **characterized in that** an amplification factor of the light-reception signal is automatically adjusted so as to contain the light-reception signal within a predetermined range.

**30.** A pulse wave measuring method according to claim 18 to 20, **characterized in that** an amount of flow of a blood is calculated based on the pulse wave.

**31.** A pulse wave measuring method according to claim 18 to 20, **characterized in that** a state of the vessel is determined based on the pulse wave, and an anesthetic depth of the patient is determined based on the state of the vessel.

**32.** A pulse wave measuring method according to claim 18, **characterized by** further comprising:

emitting a light to the vessel under a skin at a position apart from the skin;
receiving a reflected light from the vessel;
measuring a blood pressure of the patient;
determining, as a second pulse wave, a time-dependent change of the vessel based on the reflected light; and
diagnosing whether there is an abnormality due to a constriction of a coronary artery of the patient by comparing the pulse wave and the second pulse wave.

**33.** A pulse wave measuring method according to claim 18, **characterized in that** a maximum blood pressure and a minimum blood pressure are calculated based on a pulse wave area obtained from the pressure pulse wave.

**34.** A pulse wave measuring method according to claim 18, **characterized in that** an arterial cardiac output, an amount of flow of a blood, and a degree of blood oxygen saturation are serially calculated from the determined pulse wave.

**35.** A pulse wave measuring method according to claim 18, **characterized in that** if a pulse wave is not determined due to an external effect, an average value of past pulse waves is used as a substitute.

**36.** A pulse wave measuring method **characterized by** comprising:

emitting a light to a heart under a skin of a chest of a patient;
receiving a reflected light from the heart;
measuring a blood pressure of the patient; and
determining, as a pulse wave, a time-dependent change of a state of a heart based on the reflected light.

# FIG. 1

# F I G. 2

# F I G. 3

# FIG. 4

EP 1 212 979 A2

# F I G. 5A

# F I G. 5B

# F I G. 6

| Min | Max |
|-----|-----|
| 60  | 123 |
| 62  | 120 |
| 68  | 117 |
| 70  | 123 |
| ⋮   | ⋮   |

HEART RATE/MIN.

○○BEATS — H2

H1

# F I G. 7

35

CONTROL UNIT

34

31

37 38

36

33

32

P

# FIG. 8

EP 1 212 979 A2

# F I G. 9

S100 — START

S110 — INPUT CUFF PRESSURE, PRESSURE PULSE WAVES

S120 — MEASURE REFERENCE MAXIMUM/MINIMUM BLOOD PRESSURES, PULSE RATE

S130 — CALCULATE REFERENCE BLOOD PRESSURE AREA (Ax)

S140 — BODY MOTION PROCESS OF PULSE WAVES

S150 — INPUT PULSE WAVES

S160 — CALCULATE PULSE AREA (Vs)

S170 — CALCULATE AREA RATIO (Ax/Vs)

S180 — DETECT PULSE WAVES

S190 — CALCULATE CORRECTED PULSE AREA

S200 — CALCULATE MAXIMUM/MINIMUM BLOOD PRESSURES

S210 — END

# F I G. 10

# F I G. 11

# F I G. 12

BLOOD FLOW

Vs

SINGLE STROKE Tx

TIME

# F I G. 13

S300 — SET CUFF PRESSURE PICKUP

S310 — CHECK PULSE WAVE LEVEL — NO

YES

S320 — START CUFF PUMP
APPLY CUFF PRESSURE
DETECT CUFF PRESSURE
DETECT PRESSURE PULSE WAVE

S330 — CHECK CUFF PRESSURE — NO

YES

S340 — CHECK PRESSURE PULSE WAVE — NO

YES

S350 — CALCULATE REFERENCE MAXIMUM BLOOD PRESSURE VALUE, MINIMUM BLOOD PRESSURE VALUE

S360 — AQUIRE REFERENCE PULSE WAVE, REFERENCE BODY MOTION LEVEL

S370 — CALCULATE BLOOD PRESSURE COUNT FROM REFERENCE PULSEWAVE

S380 — DETECT PULSE WAVES

S390 — BODY MOTION CHECK — YES — S500

NO

S400 — CALCULATE BLOOD PRESSURE

S500 — PULSE WAVE REGENERATING PROCESS

S410 — ABNORMAL BLOOD PRESSURE? — YES

NO

S420 — OUTPUT PULSE WAVE FOR DATA DISPLAY

# FIG. 14

S510 — DETECT PULSE WAVE

S520 — BODY MOTION?
— NO → S530 — CALCULATE STROKE MASS FROM STROKE TIME, PULSE WAVE VOLUME → S540 — STORE CALCULATED VALUES OF STROKE TIME, PULSE WAVE VOLUME

— YES ↓

S550 — STROKE TIME CHECK
— NO → S560 — CONVERT STROKE TIME, PULSE WAVE LEVEL INTO AVERAGES OF PAST

— YES ↓

S570 — AVERAGE PULSE WAVE LEVEL

S580 — CALCULATE STROKE MASS

EP 1 212 979 A2

# F I G. 15

S600 — COMPLETE APPLICATION OF CUFF PRESSURE

S610 — PROCESSING OF DETECTING REFERENCE PULSE WAVE AFTER PULSE WAVES STABILIZE

S620 — DETERMINE HYPOTHETICAL FLOW SPEED OF BLOOD FLOW SPEED

S630 — CALCULATE VESSEL INSIDE WALL DIAMETER

S640 — CALCULATE RELATIVE AMOUNT OF BLOOD FLOW PER STROKE

S650 — CALCULATE CORRECTION FACTOR FROM MEASURED BLOOD FLOW PARAMETER

S660 — CALCULATE STROKE MASS, APPROXIMATE AMOUNT OF BLOOD FLOW PER STROKE

S670 — OUTPUT WAVEFORMS OF STROKE MASS, AMOUNT OF BLOOD FLOW FOR DATA DISPLAY